(19) Europäisches Patentamt
European Patent Office
Office européen des brevets

(11) Publication number: **0 214 092 A1**

(12) **EUROPEAN PATENT APPLICATION**

(21) Application number: 86810342.5

(22) Date of filing: 01.08.86

(51) Int. Cl.[4]: **A 61 K 31/47**
A 61 K 31/435, A 61 K 9/14
A 61 K 47/00
//C07D471/04

(30) Priority: 08.08.85 US 763489

(43) Date of publication of application: 11.03.87 Bulletin 87/11

(84) Designated Contracting States: BE CH DE FR GB IT LI LU NL SE

(71) Applicant: CIBA-GEIGY AG
Klybeckstrasse 141
CH-4002 Basel(CH)

(72) Inventor: Leeson, Lewis, J.
48 Pitcairn Drive
Roseland New Jersey 07068(US)

(54) **Enhanced absorption of psychoactive 2-aryl-pyrazolo quinolines as a solid molecular dispersion in polyvinylpyrrolidone.**

(57) The absorption of orally administered substantially aqueous insoluble psychoactive 2-aryl-pyrazolo [4,3-C] quinolin-3-ones of the formula

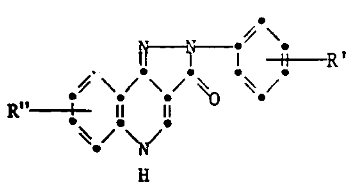

(I)

wherein R'' is hydrogen, lower alkyl, lower alkoxy, hydroxy, halo or trifluoromethyl, and R' is hydrogen, lower alkyl, lower alkoxy, hydroxy, halo, trifluoromethyl, nitro, amino, mono- or di-lower alkylamino, mono- or di-lower alkylcarbamoylamino, mono- or di-lower alkylcarbamoyloxy or cyano, or a pharmaceutically acceptable salt thereof, is enhanced by administering the same in the form of a storage stable solid molecular dispersion of polyvinylpyrrolidone.

EP 0 214 092 A1

Croydon Printing Company Ltd.

4-15457/=/CGC 1142

# ENHANCED ABSORPTION OF PSYCHOACTIVE 2-ARYL-PYRAZOLO QUINOLINES AS A SOLID MOLECULAR DISPERSION IN POLYVINYLPYRROLIDONE

## BACKGROUND OF THE INVENTION

The instant invention relates to solid molecular dispersions of one or more psychoactive and in water substantially insoluble 2-aryl-pyrazolo [4,3-C] quinolin-3-ones in polyvinylpyrolidone (PVP) exhibiting enhanced absorption upon oral administration, and a method of treating anxiety or depression in mammals by orally administering to the mammal in need thereof an effective amount of such molecular dispersion.

The psychoactive 2-aryl-pyrazolo[4,3-C]quinolin-3-ones for use in preparing the molecular dispersion having enhanced absorption characteristics are described in U.S. Patent No. 4,312,870. In general, however, the psychoactive compounds and their pharmaceutically acceptable salts exhibit poor solubility characteristics in water, gastric fluid and intestinal fluid. Also such compounds tend to exhibit irreproducible absorption characteristics upon oral administration to mammals in conventional dosage forms.

A number of studies are reported regarding the dissolution rates of various poorly water soluble drugs in various polymer dispersions. For example, Simonelli et al., J. Pharm. Sci., 58(5), 538-549 (1969), discussed the increased rate of solution of sulfathiazole from tablets wherein the sulfathiazole is previously coprecipitated with PVP. As cautioned by the author, at page 539 thereof, in order for the drug to be released from the polymer, the rate of dissolution of the drug in the solvent must be greater than the rate of dissolution in the intact polymer dispersion, otherwise the drug will remain bound as a drug-polymer complex. Moreover, the rate of drug dissolution may be restricted by the amount of free drug in solution. In the event that the free drug concentration exceeds its solubility, precipitation of the unbound drug may occur on the surface of the dissolving complex, thereby adversely affecting further drug dissolution.

Moreover, the stability of such two component solid systems is unpredictable since solid-solid phases may be unstable on storage, due to the existence of a metastable system. This problem was recognized by Allen et al., J. Pharm. Sci., 58(10), 1190-1193 (1969). Accordingly, more complicated multi-component compositions have been proposed to increase the bioavailability of poorly soluble drugs. For example, U.S. 3,862,311, issued January 21, 1975 discloses the combination of a drug, such as progesterone, a water soluble polymer, such as an ethylene oxide/propylene oxide polycondensate, and a nonionic surfactant to enhance the in-vivo absorption of the drug.

It has now been found that psychoactive 2-aryl-pyrazolo[4,3-C]quinolin-3-ones which are themselves substantially insoluble in water, gastric fluid and intestinal fluid can be formulated in pharmaceutical compositions to substantially increase the bioavailability thereof upon oral administration by providing a solid molecular dispersion thereof in polyvinylpyrrolidone.

It is an object of the instant invention to provide such compositions.

It is a further object of the instant invention to provide a method of treating a mammal by oral administration of such compositions.

It is yet a further object of the instant invention to provide methods for preparing such compositions.

These and other objects of the invention are apparent from the following detailed description of the invention.

DETAILED DESCRIPTION OF THE INVENTION

One embodiment of the invention relates to a solid oral dosage form comprising

a) an effective anxiolytic or antidepressant amount of a stable solid molecular dispersion of a compound of the formula

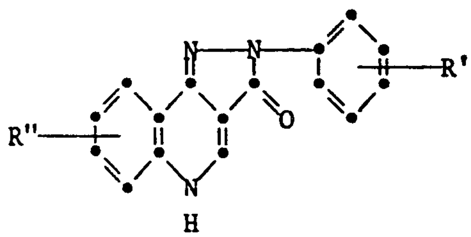

(I)

wherein R" is hydrogen, lower alkyl, lower alkoxy, hydroxy, halo, or trifluoromethyl, and R' is hydrogen, lower alkyl, lower alkoxy, hydroxy, halo, trifluoromethyl, nitro, amino, mono- or di-lower alkylamino, mono- or di-lower alkylcarbamoylamino, mono- or di-lower alkylcarbamoyloxy or cyano, or a pharmaceutically acceptable salt thereof, and

b) polyvinylpyrrolidone.

In respect to the definitions of R' and R" in the compounds of formula (I), the term "lower" in each case preferably relates to those moieties wherein the alkyl group contains 1 to 4 carbon atoms. Similarly, by "halo" is preferably meant chloro, bromo, or fluoro.

Especially preferred antidepressant compounds of formula (I) are those wherein R" is hydrogen, alkyl, or alkoxy with up to 4 carbon atoms each, hydroxy, fluoro, chloro, bromo, or trifluoromethyl; and R' is hydrogen, o- or m-fluoro; or R' is p-fluoro when R" is chloro; or a pharmaceutically acceptable salt thereof.

Such preferred antidepressant compounds also exhibit appetite suppression characteristics rendering such psychoactive compounds highly useful in the treatment of obesity and the like, see U.S. Patent No. 4,459,298, the disclosure of which is hereby incorporated by reference.

Outstanding antidepression compounds of formula (I) are those wherein R" is hydrogen or 8-(methyl, methoxy, fluoro or chloro) and R' is hydrogen, or R' is 4-fluoro when R" is 8-chloro, or a pharmaceutically acceptable salt thereof. Most preferred is the compound wherein R" and R' are both hydrogen.

Expecially preferred anxiolytic compounds of formula (I) are those wherein R" is hydrogen or 8-(methyl, methoxy, fluoro or chloro), and R' is 4-(methyl, methoxy, chloro, bromo, amino or cyano) or R' is 4-fluoro when R" is other than 8-chloro; or a pharmaceutically acceptable salt thereof. Most preferred anxiolytic compounds are those compounds wherein R" is hydrogen and R' is 4-chloro and wherein R" is 8-methoxy and R' is 4-fluoro.

Polyvinylpyrrolidone (PVP) is a synthetic polymer, which consists essentially of linear 1-vinyl-2-pyrrolidone groups, the degree of polymerization of which results in polymers of various molecular weights. The weight average molecular weight of suitable PVP polymers for use as component b) in the present invention can vary widely, but is preferably at least about 4000. Commercially available PVP having a weight average molecular weight between about 4,000 and about 1,200,000 are highly advantageous. Highly preferred are PVP polymers having a weight average molecular weight between about 8,000 and 80,000, and most preferably between about 10,000 and 70,000.

The weight ratio of the psychoactive component a) to the total molecular dispersion of component a) plus the PVP component, component b), can very widely, but is characteristically limited by the amount of psychoactive component a) capable of being molecularly dispersed within the PVP matrix so as to result in a storage-stable composition capable of providing enhanced bioavailability of component a).

Preferably, the weight percent of component a) to the molecular dispersion of component a) plus component b) is within about 0.2 to

about 20 weight percent, more preferably between about 0.5 and about 10 weight percent, and most preferably between about 0.7 and about 5 weight percent.

The solid compositions, according to the present invention, of components a) and b) are in the form of a molecular dispersion of component a) within the matrix of component b), and may exist in the form of a solid solution, a supercooled liquid solution in solid state, or a sub-colloidal dispersion of component a) within the matrix of component b). In any event, the instant molecular dispersions are clearly divergent in dissolution properties from conventional micronized formulations and the like.

The instant molecular dispersions may be conveniently prepared by dissolving PVP in water or an organic or an aqueous/organic solvent, wherein the organic solvent is a lower alkanol, such as methanol or ethanol, chloroform, dimethylsulfoxide, propylene glycol, 1-methyl-2-pyrrolidone, or the like, and adding thereto the psychoactive component a) and removing the solvent therefrom.

Conveniently, the aqueous, organic, or aqueous/organic PVP solution is maintained at an elevated temperature, e.g. between about 30°C and the boiling point of the solvent, preferably between 30°C and 60°C, and component a) is added thereto. The solution is then, after equilibration, allowed to cool, any residual precipitation of component a) is removed from the cooled solution, e.g. by filtration, and then the organic solvent is removed, e.g., by evaporation, spray drying or the like, under ambient or reduced pressure. If desired, the solid molecular dispersion obtained can subsequently be granulated. Alternatively, the solution can be dried in the form of a film or layer in a formed substrate.

Alternatively, the component a) may be simply dissolved in molten PVP and subsequently cooled to form the desired molecular dispersion.

The molten solution may be cooled by spraying or placed into forms of convenient size and shape. Alternatively, the molten solution may be cooled in bulk and subsequently granulated.

The applied dosage of the active component may range between 0.01 and 50 mg/kg/day preferably between about 0.02 and 3 mg/kg/day and advantageously between about 0.05 and 0.25 mg/kg/day.

The compositions in the form of molecular dispersions of the present invention may be incorporated into gelatin capsules or formulated into tablets, alone or in admixture with pharmaceutically acceptable excipients, including conventional diluents, lubricants, binders, disintegrants, absorbents, colorants, flavors and sweeteners.

When formulated in admixture with such excipients, the excipients are combined with the instant solid molecular dispersions by conventional mixing, granulating or coating methods respectively, and generally contain between about 0.1 to about 99% by weight, preferably between about 1 to about 60% by weight of the excipient, based on the weight of formulated composition.

The following examples, illustrating the invention, are not to be construed as limitations thereon. Temperatures are given in degrees centigrade, and all parts wherever given are parts by weights unless otherwise stated.

<u>EXAMPLE 1:</u> 40.0 g of polyvinylpyrrolidone having a weight average molecular weight of about 50,000 (USP Povidone K-30) is dissolved with stirring in absolute ethanol (USP Alcohol 3A) in an amount sufficient to result in a total solution volume of 100 ml, maintained at a temperature of about 40°. To this solution there is slowly added 1.0 g of 2-(p-chlorophenyl)-pyrazolo[4,3-C]quinolin-3(5H)-one with stirring and the resulting mixture is allowed to equilibrate with

stirring at a temperature of about 40° for a period of 7 hours. The resulting solution is then allowed to cool to ambient temperature (about 18°) over a period of about 15 hours, and is filtered through a medium porosity sintered glass funnel with partial vacuum to remove any residual undissolved material. About 20 ml of absolute ethanol is added to the filtrate, and the filtrate is poured into a 9 inch by 13-inch glass pan and evaporated under ambient conditions to dryness. A clear yellow solid glass mass results and is placed in a mortar and pulverized. The pulverized material is then groud (through a Brinkman ZM-1 grinder) to yield a fine crystalline powder consisting of a molecular dispersion of 2-(p-chlorophenyl)-pyrazolo[4,3-C]quinolin-3(5H)-one in the polyvinylpyrrolidone.

Assay results: 2.2 mg drug/100 mg total weight

EXAMPLE 2: In a manner otherwise identical to Example 1, 1.0 g of 2-(p-fluorophenyl)-8-methoxypyrazolo[4,3-C]quinolin-3(5H)-one is dissolved in 40.0 g of polyvinylpyrrolidone/absolute ethanol having a total solution volume of 100 ml, which is subsequently equilibrated, cooled, filtered, dried, pulverized and ground to yield a fine crystalline powder consisting of a molecular dispersion of the drug in polyvinylpyrrolidone containing approximately 2.5 mg drug per 100 mg total weight.

EXAMPLE 3: In a manner otherwise identical to Example 1, 1.0 g of 2-(p-phenyl)-pyrazolo[4,3-C]-quinolin-3(5H)-one is dissolved in 40.0 g of polyvinylpyrrolidone/absolute ethanol having a total solution volume of 100 ml. The mixture is subsequently equilibrated, cooled, filtered, dried, pulverized and ground to yield a fine crystalline powder consisting of a molecular dispersion of the drug in polyvinylpyrrolidone containing approximately 2.5 mg drug per 100 mg total weight.

EXAMPLE 4: In order to determine the aqueous solubility characteristics of the instant molecular dispersions, 400 mg portions of the composition prepared according to Example 1 are filled into No. 0 hard gelatin capsules to obtain dosage forms containing 8.8 mg of drug per capsule. The dissolution rate of the encapsulated material is determined by placing a capsule into 1000 ml of distilled water which is maintained at 37° with stirring. Periodically, 2 ml aliquots of solution are removed from the stirred mixture and analyzed to determine the amount of dissolved 2-(p-chlorophenyl)-pyrazolo[4,3-C]quinolin-3(5H)-one present in the stirred mixture. The tabulated results are reported in Table I.

TABLE I

| | Drug Dissolved (mg) | | |
|---|---|---|---|
| Time (min) | Run 1 | Run 2 | Average |
| 10 | 1.95 | 0.82 | 1.39 |
| 20 | 2.15 | 1.35 | 1.75 |
| 30 | 4.18 | 2.23 | 3.21 |
| 45 | 5.56 | 3.96 | 4.76 |
| 60 | 5.81 | 4.78 | 5.30 |

EXAMPLE 5: In accordance with the method of Example 1, a solid molecular dispersion containing 0.4 mg of 2-(p-chlorophenyl)-pyrazolo [4,3-C]quinolin-3(5H)-one per 100 mg polyvinylpyrrolidone having a weight average molecular weight of about 50,000 is prepared. In order to determine the bioavailability of the dispersed drug, 30 of the solid dispersion, containing 12 mg drug, is dissolved in a sufficient amount of distilled water to obtain 100 ml solution. A dosage amount based on the active drug ingredient of 1 mg per kg rat

body weight is intubated into the stomach of rats. For comparative purposes, a high surface area precipitate of 12 mg of the same drug, i.e. 2-(p-chlorophenyl)-pyrazolo[4,3-C]quinolin-3(5H)-one, is suspended in 100 ml of a solution containing 75 ml sucrose syrup USP (containing 85% sucrose in distilled water), 0.18 g Methylparaben, 0.02 g propylparaben, and a sufficient amount of distilled water to obtain 100 ml suspension is likewise intubated into the stomach of test rats in a dosage amount, based on the active drug ingredient, of 1 mg per kg rat body weight. In Table II, the results are summarized, rats 1, 2 and 3 receiving the drug/PVP solution and rats 4 and 5 receiving the drug/sucrose suspension.

TABLE II

Plasma Concentration (ng/ml) Following

Oral Administration of 1 mg/kg (Rat)

| Rat No. | 0 | 0.5 | 1 | 1.5 | 2 | 3 | 5 | 7 | AUC |
|---|---|---|---|---|---|---|---|---|---|
| 1 | 0 | 368 | 395 | 276 | 233 | 201 | 125 | 45 | 1291 |
| 2 | 0 | 408 | 303 | 275 | 225 | 131 | 111 | 62 | 1142 |
| 3 | 0 | 184 | 277 | 127 | 85 | 223 | 112 | 150 | 1066 |
| 4 | 0 | 89 | 34 | 21 | 41 | 49 | 65 | 89 | 395 |
| 5 | 0 | 54 | 34 | 438 | 26 | 37 | 39 | 101 | 517 |

* AUC represents the Area Under the Curve (0-7 hr) in ng x hr/ml.

What is claimed is:

1. A pharmaceutical composition in a storage stable solid oral dosage form having enhanced absorption properties comprising

a) an effective anxiolytic or antidepressant amount of a stable solid molecular dispersion of a compound of the formula

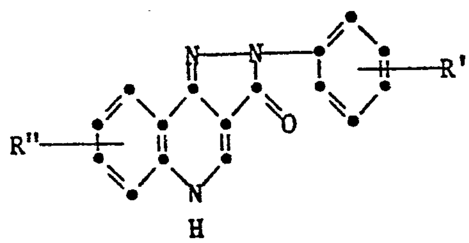

(I)

wherein R" is hydrogen, lower alkyl, lower alkoxy, hydroxy, halo, or trifluormethyl and R' is hydrogen, lower alkyl, lower alkoxy, hydroxy, halo, trifluoromethyl, nitro, amino, mono- or di-lower alkylamino, mono- or di-lower alkylcarbamoylamino, mono- or di-lower alkylcarbamoyloxy or cyano, or a pharmaceutically acceptable salt thereof and

b) polyvinylpyrrolidone.

2. A composition according to claim 1, wherein the polyvinylpyrrolidone has a weight average molecular weight of about 4,000.

3. A composition according to claim 2, wherein the ratio of component a) to the molecular dispersion of component a) plus component b) is within about 0.2 to about 20 weight percent.

4. A composition according to claim 2, wherein said weight average molecular weight is between about 4,000 and about 1,200,000.

5. A composition according to claim 4, wherein said weight average molecular weight is between about 8,000 and about 80,000.

6. A composition according to claim 3, wherein said ratio is between about 0.5 and about 10 weight percent.

7. An antidepressant composition according to claim 1, wherein R" is hydrogen or 8-(methyl, methoxy, fluoro or chloro) and R' is hydrogen or R' is 4-fluoro when R" is 8-chloro.

8. A anxiolytic composition according to claim 1, wherein R" is hydrogen or 8-(methyl, methoxy, fluoro or chloro) and R' is 4-(methyl, methoxy, chloro, bromo, amino, or cyano) or R' is 4-fluoro when R" is other than 8-chloro.

9. A composition according to claim 7, wherein R" and R' are hydrogen.

10. A composition according to claim 8, wherein R" is hydrogen and R' is 4-chloro, or R" is 8-methoxy and R' is 4-fluoro.

11. A process for the preparation of a pharmaceutical composition according to claim 1, characterised in that polyvinylpyrrolidone (PVP) is dissolved in water or an organic or an aqueous/organic solvent, the compound a) is added and the solvent is removed or that the component a) is dissolved in molten PVP and subsequently cooled.

12. Pharmaceutical compositions according to claim 1 for use in the antidepressant treatment of a human or a mammal.

13. Use of polyvinylpyrrolidone (PVP) and a compound of the formula I for the preparation of a pharmaceutical composition suitable for the antidepressant treatment of a human or a mammal.

European Patent Office

# EUROPEAN SEARCH REPORT

0214092
Application number

EP 86810342.5

| Category | Citation of document with indication, where appropriate, of relevant passages | Relevant to claim | CLASSIFICATION OF THE APPLICATION (Int. Cl.4) |
|---|---|---|---|
| D<br>Y | US - A - 4 312 870 (N. YOKOYAMA)<br>* Claims 1-5,13,15; column 4, line 25 - column 5, line 3; column 7, line 55 - column 8, line 16 *<br>-- | <br>1,7-13 | A 61 K 31/47<br>A 61 K 31/435<br>A 61 K 9/14<br>A 61 K 47/00<br>//C 07 D 471/04 |
| Y | DIE PHARMAZIE, vol. 34, no. 12, 1979, VEB Verlag Volk und Gesundheit, Berlin<br>H. KALA et al. "Anwendung der Sprühtrocknung in der Pharmazie" pages 779-784<br>* Pages 782-783, chapter 2.4.3, especially table on page 783 *<br>-- | 1,7-13 | |
| A | DIE PHARMAZIE, vol. 36, no. 2, 1981, VEB Verlag. Volk und Gesundheit, Berlin<br>H. KALA et al. "Zur Herstellung und Charakterisierung von Sprüheinbettungen"<br>pages 106-111<br>* Page 106, right column; page 107; page 108, end of left column - begin of the right column; page 110, chapter 3; page 111, chapter 4.4 *<br>-- | 1-13 | TECHNICAL FIELDS SEARCHED (Int. Cl.4)<br>A 61 K 9/00<br>A 61 K 31/00 |
| A | CH - A5 - 574 245 (BOEHRINGER MANNHEIM GMBH)<br>* Claim; example 1d *<br>-- | 1,11 | |

The present search report has been drawn up for all claims

| Place of search | Date of completion of the search | Examiner |
|---|---|---|
| VIENNA | 31-10-1986 | MAZŽUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number
0214092

EP 86810342.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE - B2 - 2 634 004 (B. LIPPOLD, R. LUTSCHG) * Claim; column 1, line 58 - column 6, last line; column 8 * -- | 1-6,11 | |
| A | GB - A - 2 119 784 (ELAN CORPORATION LTD.) * Claims 1,2,4-6; abstract * -- | 1-6,11 | |
| A | GB - A - 2 050 828 (FREUND INDUSTRIAL COMPANY LTD.) * Claims 1,2,17 * -- | 1-6,11 | |
| D,A | US - A - 4 459 298 (P.S. BERNARD) * Column 1, line 21 - column 2, line 35 * ---- | 1,7-13 | TECHNICAL FIELDS SEARCHED (Int. Cl.4) |

The present search report has been drawn up for all claims

| Place of search | Date of completion of the search | Examiner |
|---|---|---|
| VIENNA | 31-10-1986 | MAZŻUCCO |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document